# EUROPEAN PATENT APPLICATION

(11) **EP 2 389 934 A1**
(43) Date of publication of application: **30.11.2011**
(21) Application number: 11167322.4
(22) Date of filing: 24.05.2011
(51) Int. Cl.: A61K 31/197, A61K 9/20, A61K 9/00

(54) **Controlled-Release Tablet Formulations of Pregabalin**

(30) Priority: 25.06.2010 TR 201005145; 25.05.2010 TR 201004139
(71) Applicant: Sanovel Ilac Sanayi ve Ticaret A.S., 34398 Istanbul (TR)
(72) Inventor: Cifter, Ümit, 34398, Istanbul (TR); Türkyilmaz, Ali, 34398, Istanbul (TR); Akalin, Nur Pehlivan, 34398, Istanbul (TR)
(74) Representative: Sevinç, Erkan

(57) **Abstract**

The present invention relates to a controlled-release tablet formulation which gels in the stomach, comprising pregabalin or a pharmaceutically acceptable salt thereof, and a complex of sodium alginate and calcium chloride, or calcium alginate, or a properly-proportioned mixture thereof as a controlled-release agent.

## Description

The present invention relates to pharmaceutical compositions of pregabalin or a pharmaceutically acceptable salt thereof. The present invention more particularly relates to a stable controlled-release formulation of pregabalin, with a release profile of desired efficiency.

### Background of Invention

Pregabalin is an analog of gamma-aminobutyric acid (GABA). Its chemical designation is (S)-3-(aminomethyl)-5-methyl hexanoic acid, with the chemical structure illustrated below in Formula 1.

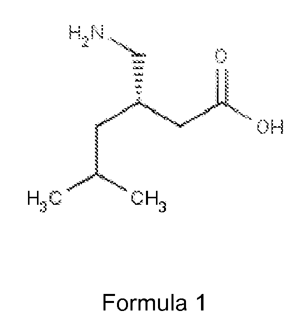

It is known that pregabalin binds to the auxiliary subunit of voltage-sensitive calcium channels in the central nervous system, thereby replacing [3H]-gabapentin. It also reduces the release of many neurotransmitters, including pregabalin glutamate, noradrenaline, and the substance P. It is used for the treatment of epilepsy, simple or complex partial convulsion, either accompanied or not by secondary generalized convulsions, and of neuropathic pain. Various formulations of pregabalin have been developed. It is generally provided in the form of conventional capusel formulations. Posology requires such formulations to be administered twice or thrice daily.

Various applications can be found in relation to pregabalin in the patent literature.

Patent application W02008008120, for instance, discloses a solid dosage form comprising a compacted fill material containing at least one active agent and at least one of a disintegrating agent and wetting agent.

Patent application W02007052125 claims a pharmaceutical composition containing pregabalin or a pharmaceutically acceptable complex, salt, solvate, or hydrate thereof, and excipients such as a matrix forming agent and a swelling agent.

Patent application W02008140459, in turn, discloses a solid dosage form comprising a compacted fill material having a pressure-sensitive multi-particulate and at least one cushioning agent. Said multi-particulate and/or cushioning agent comprises at least one active agent and display(s) a weight loss less than 1% in 30 minutes according to the friability test USP 29 test # 1216. The compacted fill material has a density of at least 0.5 g/ml and a tensile strength of less than 0.9 MPa.

Patent application W02008140460, on the other hand, claims a solid dosage form comprising a compacted fill material including at least one active agent. The solid dosage form displays a weight loss of less than 1% in 30 minutes in accordance with the friability test USP 29 test # 1216. Compacted fill material particles contain at least one active agent in the matrix and provide a controlled release of the active agent.

Patent application W02008128775 claims a solid pharmaceutical composition comprising pregabalin as an active agent, together with one or more excipients. Said composition is free from saccharides and comprises no further amino acids.

Patent application W02009066325 provides a controlled release formulation comprising pregabalin or a pharmaceutically acceptable salt thereof, a hydrophobic release agent, and an excipient.

Stability-related problems do occur in a plurality of active agents, including pregabalin, under the influence of ambient and physical conditions. As disclosed in patent W09959573, pregabalin, an amino acid derivative, undergoes cyclization and converts into a lactam form, even if normal storage conditions are provided. This is not desirable for the formulation.

Pregabalin is an active agent with quite good solubility and dissolution rate. This fact leads to fluctuations in the release profile of controlled-release formulations aimed to be developed. Such fluctuations, in turn, brings about imbalances in the blood plasma levels of active agent, and resultantly, undesired effects are produced on the subject.

Another problematic situation for pregabalin, in fact, is its absorption in the gastrointestinal tract. It has been observed that pregabalin absorption increases from small intestine towards large intestine, and becomes poor beyond the hepatic flexure. Conventional tablets are transferred to the hepatic flexure in about 6 hours or less, on average, thereafter loosing efficiency due to poor absorption in the remaining parts of the intestine.

In result, the aforesaid drawbacks require a novelty in the art of pregabalin formulations with antiepileptic, analgesic, and anxiolytic activities.

### Object and Brief Description of Invention

The present invention relates to a controlled-release pregabalin formulation, gelling in the stomach and floating in gastric juice, thereby eliminating all aforesaid problems and brining additional advantages to the relevant prior art.

Accordingly, the main object of the present invention is to obtain a stable formulation with antiepileptic, analgesic, and anxiolytic activities.

Another object of the present invention is to provide a pregabalin formulation administered orally once or twice per day.

A further object of the present invention is to ensure a high drug-absorption by retarding the advancement of said formulation through the gastrointestinal tract, thanks to a pregabalin formulation that gels in the stomach and floats in gastric juice.

Yet a further object of the present invention is to provide a stable formulation by preventing pregabalin used in the subject formulation from converting into the lactam form via cyclization.

Still a further object of the present invention is to embody a controlled-release formulation with uniform release profile.

A controlled-release tablet formulation, gelling in the stomach and floating in gastric juice, has been developed to carry out any objects, referred to above and to emerge from the following detailed description.

In a preferred embodiment according to the present invention, said novelty is carried out with pregabalin or a pharmaceutically acceptable salt thereof, and a complex of sodium alginate and calcium chloride, or calcium alginate, or a properly-proportioned mixture thereof, for use as a controlled-release agent.

In a preferred embodiment according to the present invention, polycarbophil is also included as the controlled-release agent.

In a preferred embodiment according to the present invention, the proportion of polycarbophil to the total tablet weight is 0.01 to 2%.

In a preferred embodiment according to the present invention, the proportion of pregabalin to the total weight of the complex of sodium alginate and calcium chloride complex, or of calcium alginate, or of a properly-proportioned mixture thereof is between 0.01 to 20, preferably 0.05 to 16, and more preferably 0.1 to 15.

In a preferred embodiment according to the present invention, the proportion of sodium alginate to calcium chloride in the complex of sodium alginate and calcium chloride is 2:1.

In a preferred embodiment according to the present invention, excipients are included, comprising at least one or a mixture of glidants and lubricants.

In a preferred embodiment according to the present invention, said glidants comprise at least one or a mixture of colloidal silicone dioxide, talk, magnesium carbonate, calcium stearat, aluminum silicate, magnesium silicate, with colloidal silicone dioxide being preferred.

In a preferred embodiment according to the present invention, magnesium stearate is included as the lubricant.

In a preferred embodiment of the present invention, said gel formulation floats in gastric juice.

Another preferred embodiment according to the present invention provides a method for preparing said pharmaceutical formulation, this method comprising the steps of
a. admixing pregabalin, together with colloidal silicone dioxide, and sieving the resulting mixture,
b. adding calcium alginate or a complex of sodium alginate and calcium chloride to this sieved powder mixture, and mixing the resulting mixture,
c. adding magnesium stearate into the final mixture, mixing the resulting mixture and compacting it in the form of tablets.

A further preferred embodiment according to the present invention provides a method for preparing said pharmaceutical formulation, this method comprising the steps of
a. admixing pregabalin, together with calcium alginate or a mixture of sodium alginate and calcium chloride,
b. wet granulating the mixture with water or water-alcohol (hydroalcoholic solution),
c. drying the wet granules in a drier and sieving the same,
d. adding colloidal silicone dioxide and magnesium stearate to the sieved granules, mixing the resulting mixture, and compacting it in the form of tablets.

Another preferred embodiment according to the present invention provides a method for preparing said pharmaceutical formulation, this method comprising the steps of
a. admixing pregabalin, together with calcium alginate or a mixture of sodium alginate and calcium chloride,
b. compacting the mixture obtained above via pre-compaction or compactor,
c. adding colloidal silicone dioxide and magnesium stearate to the sieved powder mixture, mixing the resulting mixture, and compacting it in the form of tablets.

In another preferred embodiment according to the present invention, said pharmaceutical formulation is consisted of the following ingredients only:
a. pregabalin or a pharmaceutically acceptable salt thereof at 25-75% by weight
b. complex of sodium alginate and calcium chloride, or calcium alginate, or a properly-proportioned mixture thereof at 5-90% by weight,
c. colloidal silicone dioxide at 0.1 to 5% by weight,
d. magnesium stearate at 0.1-5% by weight.

### Detailed Description of Invention

### Example 1:

| **Ingredient** | **amount %** |
|---|---|
| pregabalin | 25 - 75 |
| complex of sodium alginate and calcium chloride, or calcium alginate | 5 - 90 |
| colloidal silicone dioxide | 0.25 - 2 |
| magnesium stearate | 0.25 - 2 |

### Example 2:

| **Ingredient** | **amount %** |
|---|---|
| pregabalin | 25 - 75 |
| complex of sodium alginate and calcium chloride, or calcium alginate | 5 - 90 |
| polycarbophil | 0.5 - 2 |
| colloidal silicone dioxide | 0.25 - 2 |
| magnesium stearate | 0.25 - 2 |

Various production methods can be applied for the controlled-release formula that gels in the stomach, with the formulations indicated in the examples above.

In the direct compaction method, pregabalin is admixed together with colloidal silicone dioxide, and the resulting mixture is sieved. To this sieved powder mixture, calcium alginate or a complex of sodium alginate and calcium chloride is added, and the resulting mixture is mixed. Magnesium stearate is added to the final mixture, the resulting mixture is mixed and is compacted into tablets.

In the wet granulation production method, pregabalin is admixed together with calcium alginate or a mixture of sodium alginate and calcium chloride. The mixture is wet granulated with water or water-alcohol (hydroalcoholic solution). After the wet granules are dried in a drier, they are sieved, and to the sieved granules are added colloidal silicone dioxide and magnesium stearate, the resulting mixture is mixed and compacted in the form of tablets.

In another production method, pregabalin is admixed together with calcium alginate or a mixture of sodium alginate and calcium chloride. The mixture is compacted via pre-compaction or compactor and is then sieved. To this sieved powder mixture, colloidal silicone dioxide and magnesium stearate are added, the resulting mixture is mixed and is compacted into tablets.

### Example 3

| **Ingredient** | **amount %** |
|---|---|
| pregabalin | 38,5 |
| sodium/calcium alginate | 23,9 |
| dibasic calcium phosphate | 15,9 |
| colloidal silicone dioxide (large surface area ) | 21 |
| colloidal silicone dioxide | 0,1 |
| magnesium stearate | 0,7 |
| **Film coating** | |
| Opadry AMB/kollicoat IR | 3-5 |

In the direct compression production, pregabalin , sodium/calcium alginate, dibasic calcium phosphate, colloidal silicone dioxide with large surface area are mixed together. Then mixture is added colloidal silicone dioxide and magnesium stearate. Finally, the mixture is compressed into tablets and coated.

Larged surface area Colloidal silicone dioxides are Aeropearl 300 (300m2/g) and Aerosil 380 (380m2/g))

### Example 4

| **Ingredient** | **amount %** |
|---|---|
| pregabalin | 44 |
| sodium/calcium alginate | 12,9 |
| hydroxypropyl methyl cellulose | 12 |
| dibasic calcium phosphate | 19,5 |
| polypropylene | 10,7 |
| colloidal silicone dioxide | 0,1 |
| magnesium stearate | 0,8 |
| **Film coating** | |
| Opadry AMB/kollicoat IR | 3-5 |

In the wet granulation production, pregabalin, polypropylene, hydroxypropyl methyl cellulose and dibasic calcium phosphate are mixed together. Sodium/calcium alginate is granulated with hydroalcoholic solution and then dried and sieved. Then granules are added colloidal silicone dioxide and magnesium stearate and then are mixed. Finally, the mixture is compressed into tablets and coated.

This invention has surprisingly provided a controlled-release pregabalin tablet formulation, which gels in the stomach and floats in gastric juice, as well as is stable and has a desired release profile. The formulation according to the present invention swells upon contact with gastric juice, thereby decreasing its density and floating in gastric juice. Thanks to this fact, the advancement of the formulation through the gastrointestinal tract is retarded. Thus, the advancement of the pregabalin-containing formulation through the gastrointestinal tract is delayed and its absorption is made to occur at a site in which adsorption takes place more efficiently. An ideal absorption of pregabalin occurs only at a certain site of small intestine. Retaining the drug at an efficient absorption site enhances its bioavailability. In contrast to most other polysaccharide gels, alginate gels can develop instantaneously in the presence of divalent cations into acid gels at low pH and constant temperature. The gelling gives rise to a three dimensional network which determines the gel strength.

It is possible to make various controlled-release formulations containing pregabalin. For instance, it is possible to develop formulations which are retained in the stomach so that their advancement is delayed, or which are liquid out of the body and gel in the stomach with a volume increase. These systems, for instance, can float within stomach due to their low densities. Various excipients can be used for this purpose, such as alginates (salts thereof), gums, oil, gelling agents.

The present invention is used for treating epilepsy, pain, anxiety, diabetic neuropathy, neuropathic pain, partial seizure, social phobia, postherpetic neuralgia.

It is further possible to use the following additional excipients in the formulation.

The pharmaceutical compositions according to the present invention may also comprise one or more pharmaceutically acceptable excipients. Such pharmaceutically acceptable excipients include, but are not restricted to fillers, binders, glidants, lubricants, disintegrants, surface active agents, preserving agents, coating agents etc., and the mixtures thereof.

Suitable binders include, but are not restricted to, at least one or a mixture of polyvinylprolidone, gelatin, sugars, glucose, natural gums, gums, synthetic celluloses, polymethacrylate, hydroxypropyl methyl cellulose, hydroxypropyl cellulose, carboxymethyl cellulose, methyl cellulose, and other cellulose derivatives.

Suitable lubricants include, but are not restricted to, at least one or a mixture of sodium stearil fumarat, polyethylene glycol, stearic acid, metal stearates, boric acid, sodium chloride benzoate and acetate, sodium or magnesium lauryl sulfate.

Suitable coating agents include, but are not restricted to hydroxypropyl methyl cellulose, polyethylene glycol, polyvinylprolidone, polyvinylprolidone-vinyl acetate copolymer (PVP-VA), polyvinyl alcohol like polymers, and all sorts of Opadry^{™}, as well as pigments, dyes, titanium dioxide and iron oxide, talk.

The present invention is hereby disclosed by referring to exemplary embodiments hereinabove. Whilst this exemplary embodiment does not restrict the object of the present invention, the latter must be assessed under the light of the foregoing detailed description.

## Claims

1. A controlled-release tablet formulation which gels in the stomach, comprising pregabalin or a pharmaceutically acceptable salt thereof, and a complex of sodium alginate and calcium chloride, or calcium alginate, or a properly-proportioned mixture thereof as a controlled-release agent.

2. The pharmaceutical formulation according to Claim 1, further comprising polycarbophil as the controlled-release agent.

3. The pharmaceutical formulation according to any of the preceding claims, wherein the proportion of polycarbophil to the total weight of the tablet is 0.01 to 2%.

4. The pharmaceutical formulation according to any of the preceding claims, wherein the proportion of pregabalin to the total weight of the complex of sodium alginate and calcium chloride complex, or of calcium alginate, or of a properly-proportioned mixture thereof is between 0.01 to 20, preferably 0.05 to 16, and more preferably between 0.1 to 15.

5. The pharmaceutical formulation according to any of the preceding claims, wherein the proportion of sodium alginate to calcium chloride in the complex of sodium alginate and calcium chloride is 2:1.

6. The pharmaceutical formulation according to any of the preceding claims, further comprising at least one or a mixture of glidants and lubricants as an excipients.

7. The pharmaceutical formulation according to any of the preceding claims, **characterized in that** said glidant comprises at least one or a mixture of colloidal silicone dioxide, talk, magnesium carbonate, calcium stearate, aluminum silicate, and magnesium silicate, said glidant being preferably colloidal silicone dioxide.

8. The pharmaceutical formulation according to any of the preceding claims, comprising magnesium stearate as the glidant.

9. A method for preparing a pharmaceutical formulation according to any of the preceding claims, comprising the steps of
a. admixing pregabalin, together with colloidal silicone dioxide, and sieving the resulting mixture,
b. adding calcium alginate or a complex of sodium alginate and calcium chloride to this sieved powder mixture, and mixing the resulting mixture,
c. adding magnesium stearate into the final mixture, mixing the resulting mixture and compacting it into tablets.

10. A method for preparing a pharmaceutical formulation according to any of the preceding claims, comprising the steps of
a. admixing pregabalin, together with calcium alginate or a mixture of sodium alginate and calcium chloride,
b. wet granulating the mixture with water or water-alcohol (hydroalcoholic solution),
c. drying the wet granules in a drier and sieving the same,
d. adding colloidal silicone dioxide and magnesium stearate to the sieved granules, mixing the resulting mixture, and compacting it into tablets.

11. A method for preparing a pharmaceutical formulation according to any of the preceding claims, comprising the steps of
a. admixing pregabalin, together with calcium alginate or a mixture of sodium alginate and calcium chloride,
b. compacting the mixture via pre-compaction or compactor, then sieving the same,
c. adding colloidal silicone dioxide and magnesium stearate to the sieved powder mixture, mixing the resulting mixture, and compacting it into tablets.

12. The pharmaceutical formulation according to any of the preceding claims, consisting of,
a. pregabalin or a pharmaceutically acceptable salt thereof at 25-75% by weight
b. complex of sodium alginate and calcium chloride, or calcium alginate, or a properly-proportioned mixture thereof at 5-90% by weight,
c. colloidal silicone dioxide at 0.1 to 5% by weight,
d. magnesium stearate at 0.1-5% by weight.

13. A pharmaceutical formulation according to any of the preceding claims for use in the prevention or treatment of epilepsy, pain, anxiety, diabetic neuropathy, neuropathic pain, postherpetic neuralgia in mammalians, particularly in humans

14. The pharmaceutical formulation according to any of the preceding claims, this formulation being floatable in gastric juice.
